# EUROPÄISCHE PATENTANMELDUNG

(11) **EP 0 640 316 A1**
(43) Veröffentlichungstag der Anmeldung: **01.03.1995**
(21) Anmeldenummer: 94112565.0
(22) Anmeldetag: 11.08.1994
(51) Int. Cl.: A61B 17/22, A61B 17/36

(54) **Sonde zum intrakoporalen Fragmentieren von Steinen**

(30) Priorität: 25.08.1993 DE 9312684 U
(71) Anmelder: Richard Wolf GmbH, D-75438 Knittlingen (DE)
(72) Erfinder: Bonnet, Ludwig, D-75438 Knittlingen (DE); Lamprecht, Michael, D-76199 Karlsruhe (DE); Schmeller, N., Dr., Klinikum Grosshadern, D-81377 München (DE); Vorreuther, Dr. R., Klinik und Poliklinik für, D-50931 Köln (DE)
(74) Vertreter: Wilcken, Thomas, Dipl.-Ing.

(57) **Zusammenfassung**

Bei einer Sonde (1) zum intrakorporalen Fragmentieren von Steinen (3), insbesondere von in Harnleitern und Gallengängen befindlichen Steinen, wird mit elektrohydraulischen Stoßwellen (10) gearbeitet, die durch Funkenüberschlag zwischen Polen (8, 9) am distalen Sondenende erzeugt werden. Die Pole (8, 9) sind von einer Abschirmung (11) umgeben, die eine radiale Ausbreitung der Stoßwellen (10) verhindern soll.

## Beschreibung

Die Erfindung betrifft eine Sonde zum intrakorporalen Fragmentieren von Steinen, insbesondere von in Harnleitern und Gallengängen befindlichen Steinen, mit elektrohydraulischen Stoßwellen, die durch Funkenüberschlag zwischen Polen am distalen Sondenende erzeugt werden.

Solche sogenannten EHL-Sonden haben zwei elektrische Leiter, die koaxial oder nebeneinander durch die Sonde verlaufen und deren distale Enden Pole bzw. Elektroden für die Funkenstrecke bilden. Proximal werden die isolierten Leiter an eine Spannungsquelle angeschlossen, so daß bei Beaufschlagung der Leiter mit einem Spannungsimpuls zwischen den Polen ein Funkenüberschlag stattfindet, der eine sich kugelförmig ausbreitende Stoßwelle in dem den Stein umgebenden Medium erzeugt.

Eine Kugelwelle kann beispielsweise bei der Bearbeitung von Steinen in Harnleitern dazu führen, daß ein erheblicher Teil, vor allem der sich unmittelbar am Sondenende radial ausbreitende Teil der Stoßwelle, direkt auf die Harnleiterwand trifft und diese unter ungünstigen Umständen beschädigt oder sogar perforiert, was zu schwerwiegenden Folgen für den Patienten führen und einen erneuten Eingriff zur Behebung des Defektes erforderlich machen kann.

Im übrigen geht verständlicherweise auch der von der Sondenspitze im wesentlichen radial abgestrahlte Teil der Stoßwelle ungenutzt verloren, so daß insgesamt gesehen mehr Stoßwellenenergie zur Verfügung stehen muß als eigentlich nur für die Zerstörung des Steines erforderlich ist.

Die Aufgabe der Erfindung besteht in der Vermeidung der aufgezeigten Nachteile. Dementsprechend soll eine EHL-Sonde geschaffen werden, die eine hohe Effektivität hat und bei deren Anwendung die Gefahr einer Traumatisierung oder Perforierung von Gewebe ausgeschlossen oder zumindest weitgehend reduziert ist.

Diese Aufgabe wird ausgehend von einer Sonde der eingangs erwähnten Art erfindungsgemäß so gelöst, daß die Pole von einer die radiale Ausbreitung der Stoßwelle verhindernden Abschirmung umgeben sind.

Die Abschirmung kann durch eine distalwärts über die Pole hinaus vorstehenden Hülse gebildet sein, die auf dem distalen Endbereich der Sonde sitzt.

Durch diese Abschirmung bzw. Hülse, die am Sondenende einen Überstand bildet, wird die seitliche Ausbreitung der Stoßwelle und damit die Perforationsgefahr auf ein unbedenkliches Maß reduziert und weiterhin erreicht, daß die von innen auf die Abschirmung treffenden Stoßwellenanteile reflektiert werden, wodurch eine keilförmige Bündelung der Stoßwelle erfolgen wird.

Aufgrund dieser Bündelung der Stoßwelle wird außerdem die verfügbare Energie effektiver ausgenutzt und vor allem bei harten Konkrementen ein besseres Desintegrationsergebnis erzielt. Bedingt durch die Bündelung der Stoßwelle werden am Konkrement zwar kleine, aber wesentlich tiefere Krater als bei Anwendung herkömmlicher Sonden geschaffen, bei denen durch die größere Streuung der Stoßwelle ein wesentlich schlechterer Abtragungseffekt erzielt wird.

Wegen der höheren Effektivität der erfindungsgemäßen Sonde können auch die abgegebene Stoßwellenenergie und damit die Gefahr einer Traumatisierung oder Perforierung des Gewebes reduziert werden. Auch wird ein direkter Funkendurchschlag durch das Gewebe etwa bei tangentialer Berührung des Gewebes mit der Sonde verhindert, was besonders in engen Harnleitern einen großen Vorteil darstellt.

Im übrigen besteht die Möglichkeit, die axiale Länge der Abschirmung zu verändern, um so zu einer mehr oder weniger großen Bündelung der abgegebenen Stoßwelle und Abschirmung des gefährdeten Gewebes zu kommen. Zu diesem Zweck kann die erwähnte Hülse beispielsweise längs auf der Sonde verstellt werden.

In der anliegenden Zeichnung sind Ausführungsbeispiele der Erfindung dargestellt. Es zeigt:
- Figur 1: den distalen Bereich einer durch ein Endoskop in einen Körperkanal eingeführten Sonde,
- Figur 2: ein distales Sondenende in größerem Maßstab und
- Figur 3: ein anderes als in Figur 2 ausgebildetes distales Sondenende.

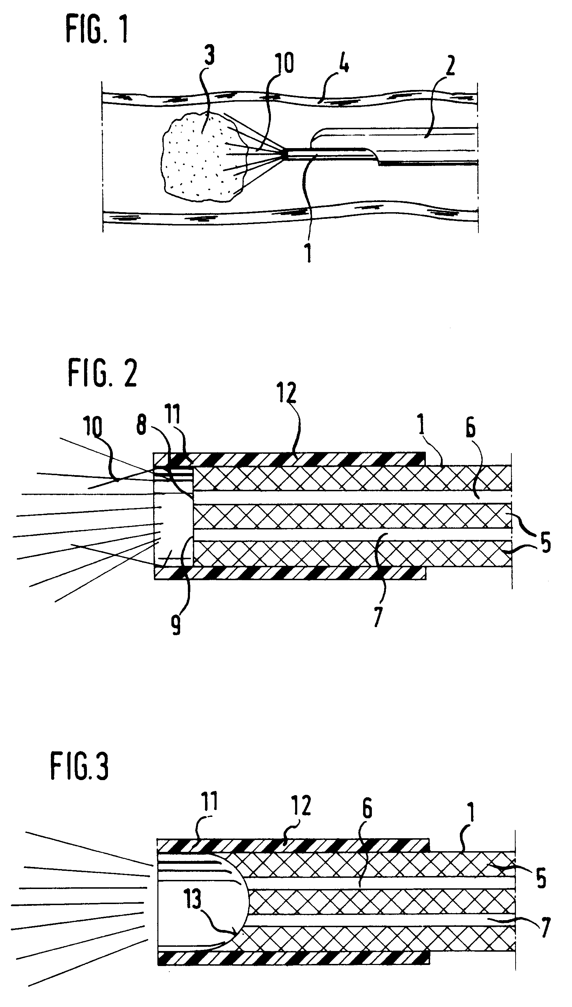

Die Sonde 1 befindet sich gemäß Figur 1 in einem Instrumentenkanal eines Endoskopes 2 und wird mit ihrem Ende unter Beobachtung über eine Optik des Endoskopes zusammen mit diesem an einen zu zerstörenden Stein 3 herangeführt, der sich beispielsweise in einem Harnleiter 4 befindet.

Die Sonde 1 hat zwei nebeneinander in einer Isolierung 5 verlaufende elektrische Leiter 6, 7, die in bekannter Weise proximal an eine nicht gezeigte Spannungsquelle angeschlossen sind und deren distale Enden freiliegende Elektroden bzw. Pole 8, 9 darstellen, deren Abstand zueinander die Länge einer Funkenstrecke bestimmt, über die beim Anlegen eines Spannungsimpulses an die Leiter 6, 7 ein Funkenüberschlag mit der Folge einer Stoßwelle 10 erzeugt wird.

Eine radiale Ausbreitung dieser Stoßwelle unmittelbar am freien Sondenende wird nach der Erfindung durch eine die Pole 8, 9 umgebende Abschirmung 11 verhindert. Hierzu ist gemäß Figur 2 eine auf die Sonde 1 geschobene Hülse 12 vorgesehen, die distalwärts gesehen über die Pole 8, 9 hinaus vorsteht und so einen Überstand als Abschirmung bildet, an der die Stoßwelle anteilig reflektiert wird und dieser Anteil nicht direkt auf die Harnleiterwand treffen kann.

Die Hülse 12 kann bei Bedarf und zwecks Änderung der wirksamen Länge des Überstandes bzw. der Abschirmung und zwecks Einstellung der Bündelung der Stoßwelle auf der Sonde 1 axial beispielsweise durch Verschieben verstellt werden. Wenn eine als günstig ermittelte Länge der Abschirmung fest vorgegeben werden kann, besteht auch die Möglichkeit, an der stirnseitigen Fläche der Isolierung 5 einen Ring anzuformen oder zu befestigen, der die Pole 8, 9 als Abschirmung umläuft.

Bei der Sonde gemäß Figur 2 ist die Hülse 12 ein Zylinder, so daß auch die durch das freie Hülsenende gebildete Abschirmung zylindrisch ist. Eine besonders effektive Abschirmung und Bündelung der Stoßwelle läßt sich auch erreichen, wenn das innere distale Sondenende gemäß Figur 3 eine rotationssymmetrische, räumlich gekrümmte Oberfläche 13 hat, die zusammen mit der Abschirmung 11 als Reflektor für die radialen Anteile der Stoßwelle wirkt.

Zu diesem Zweck kann das innere Sondenende eine konkave Oberfläche 13 haben, deren Scheitel zwischen den Polen 8, 9 liegt. Im übrigen könnte in diesem Fall die Hülse 12 entfallen, wenn der distalwärts vorstehende Randbereich des Sondenendes ausreichend lang ist und die erforderliche Abschirmung bilden kann und wenn eine Abschirmung mit fest vorgegebener Länge den gestellten Anforderungen genügen wird.

## Patentansprüche

1. Sonde (1) zum intrakorporalen Fragmentieren von Steinen (3), insbesondere von in Harnleitern und Gallengängen befindlichen Steinen, mit elektrohydraulischen Stoßwellen (10), die durch Funkenüberschlag zwischen Polen (8, 9) am distalen Sondenende erzeugt werden, dadurch gekennzeichnet, daß die Pole (8, 9) von einer die radiale Ausbreitung der Stoßwelle (10) verhindernden Abschirmung (11) umgeben sind.

2. Sonde nach Anspruch 1, dadurch gekennzeichnet, daß die Abschirmung (11) durch eine distalwärts über die Pole (8, 9) hinaus vorstehende Hülse (12) gebildet ist.

3. Sonde nach Anspruch 2, dadurch gekennzeichnet, daß die Hülse (12) auf dem distalen Endbereich der Sonde (1) sitzt.

4. Sonde nach einem der Ansprüche 1 bis 3, dadurch gekennzeichnet, daß die axiale Länge der Abschirmung (11) veränderbar ist.

5. Sonde nach einem der Ansprüche 2 bis 4, dadurch gekennzeichnet, daß die Hülse (12) längs verstellbar ist.

6. Sonde nach einem der Ansprüche 1 bis 5, dadurch gekennzeichnet, daß die Abschirmung (11) zylindrisch ausgebildet ist.

7. Sonde nach einem der Ansprüche 1 bis 6, dadurch gekennzeichnet, daß das distale Sondenende eine rotationssymmetrische, räumlich gekrümmte Oberfläche (13) als Reflektor hat.

8. Sonde nach Anspruch 7, dadurch gekennzeichnet, daß das distale Sondenende eine konkave Oberfläche (13) hat, deren Scheitel zwischen den Polen (8, 9) liegt.
